# EUROPEAN PATENT APPLICATION

(11) **EP 3 973 976 A1**
(43) Date of publication of application: **30.03.2022**
(21) Application number: 19929806.8
(22) Date of filing: 22.05.2019
(51) Int. Cl.: A61K 36/82, A61P 31/14, A61P 31/16, A23L 33/105

(54) **PHARMACEUTICAL COMPOSITION COMPRISING EXTRACT FROM CAMELLIA JAPONICA AS ACTIVE INGREDIENT FOR PREVENTION AND TREATMENT OF VIRAL INFECTION**

(71) Applicant: Huscion Co., Ltd., Seongnam-si, Gyeonggi-do 13488 (KR)
(72) Inventor: JOO, Seong Soo, Yongin-Si Gyeonggi-do 17100 (KR)
(74) Representative: Eisenführ Speiser
(86) International application number: PCT/KR2019/006129
(87) International publication number: WO 2020/235717

(57) **Abstract**

The present invention provides a pharmaceutical composition containing *Camellia japonica* extract derived from a natural substance as an active ingredient for prevention and treatment of viral infection, wherein the extract inhibits viral replication and release from cells to effectively regulate viral infection, thus exhibiting an excellent antiviral effect.

<110>    GANGNEUNG-WONJU NATIONAL UNIVERSITY INDUSTRY ACADEMY COOPERATION
GROUP

<120>    Composition for treating and preventing viral infection
         comprising extracts from Camellia japonica

<130>    PT19-5188

<160>    2

<170>    KoPatentIn 3.0

<210>    1
<211>    20
<212>    DNA
<213>    Artificial Sequence

<220>
<223>    Forward


<400>    1
tacagcttca ccaccacagc                                                    20


<210>    2
<211>    20
<212>    DNA
<213>    Artificial Sequence

<220>
<223>    Reverse


<400>    2
aaggaaggct ggaaaagagc                                                    20

## Description

### Technical Field

The present disclosure relates to a pharmaceutical composition for prevention and treatment of a viral infection and, more specifically, to a pharmaceutical composition containing a *Camellia japonica* extract as an active ingredient for prevention and treatment of a viral infection.

### Background Art

Influenza is an acute respiratory disease caused by an influenza virus infection, and in Korea, an epidemic of influenza occurs mostly in winter. Such influenza viruses are single-chain RNA viruses belonging to the Orthomyxovirus family, and influenza viruses are classified into Influenza A, B, and C viruses according to the antigen type, and of these, influenza A viruses have various mutations, causing global pandemic from which many people suffer. Retroviruses are also a type of RNA virus, and have reverse transcriptase for inserting RNA containing its own genetic information into DNA of a host cell. Due to these characteristics, most RNA viruses do not follow the central dogma. A capsid protein enclosing viral RNA is composed of a proteinaceous outer membrane for infiltrating into an adjacent host cell when releasing from the host cell. The viruses are characterized in that DNA is synthesized by using RNA as a template together with such reverse transcriptase. RNA viruses, unlike general DNA enzymes, have no ability to correct genetic errors during synthesis, resulting in various viral mutants. Like general viruses, each RNA virus itself infiltrates into a host cell to become a part of the host, and therefore, it is impossible for the immune system of the body to remove only the virus, and when a strong immune system attacks a virus-infected host cell, the host cell is also destroyed, resulting in damages to the functions of body organs. Hence, for the fundamental treatment of a viral infection, a method of primarily inhibiting a viral infection and a method of attacking and selectively destroying a virus-infected cell are effective. A human immunodeficiency virus may be caused by a retrovirus infecting humans. In this regard, Korean Patent Publication No. 2016-0024092 discloses a pharmaceutical composition containing a *Camellia japonica* extract or an oleanane triterpene derivatives separated therefrom for prevention or treatment of a corona virus-related diseases.

### Disclosure of Invention

### Technical Problem

However, the prior art has a problem that, due to the compound derivative, side effects such as drug resistance may occur and the antiviral effect is limited.

An aspect of the present disclosure is to provide a pharmaceutical composition for prevention and treatment of a viral infection, the pharmaceutical composition containing as an active ingredient a *Camellia japonica* extract derived from a natural product and exhibiting an antiviral effect by inhibiting the replication and egression of the virus. However, these problems are exemplary, and the scope of the present invention is not limited thereto.

### Solution to Problem

In accordance with an aspect of the present disclosure, there is provided a pharmaceutical composition containing a *Camellia japonica* extract or an active fraction separated by chromatography of the *Camellia japonica* extract as an active ingredient for prevention and treatment of a viral infection.

### Advantageous Effects of Invention

According to an embodiment of the present disclosure, the production effect of a pharmaceutical composition for prevention and treatment of a viral infection, which contains as an active ingredient a *Camellia japonica* extract derived from a natural product and exhibits an antiviral effect by inhibiting the replication and egression of the virus to effectively control the infection with the virus can be implemented. The scope of the present disclosure is not limited by such an effect.

### Brief Description of Drawings

FIG. 1 is a schematic diagram showing a manufacturing process for a *Camellia japonica* leaf hot-water extract of the present disclosure.
FIG. 2 is a graph showing the separation and fractionation conditions for the *Camellia japonica* leaf hot-water extract of the present disclosure.
FIG. 3 is a gel image showing the TLC analysis results of the *Camellia japonica* leaf hot-water extract and the F2 fraction of the present disclosure.
FIG. 4 is a gel image of PCR analysis showing the inhibitory effect on actin gene expression in the Raw264.7 cell line according to the treatment with the *Camellia japonica* leaf hot-water extract of the present disclosure. Comparison was made by the treatment with 1 mM hydrogen peroxide as a control.
FIG. 5 is a gel image of PCR analysis showing the inhibitory effects of reverse transcriptase activity and DNA polymerase activity in the Raw264.7 cell line according to the treatment with the *Camellia japonica* hot-water extract of the present disclosure.
FIG. 6 is a gel image showing the actin gene PCR analysis results after the treatment with the *Camellia japonica* extract (CWE) and the CWE fractions (F1-F4) in the Raw264.7 cell line according to the treatment with the *Camellia japonica* hot-water extract of the present disclosure. *CWE: 100 µg/mL, F1-F4: 50 µg/mL.
FIG. 7 is an immunocytochemical staining analysis results of actin protein after the treatment with the CWE fraction (F2) of the present disclosure at different concentrations in the Raw264.7 cell line.
FIG. 8 is a gel image showing the viral gene (pQCXIP) PCR analysis results after the treatment with CWE and the fraction (F2) in the GP2-293 packaging cells. *CWE: 100 µg/mL, F2: 50 µg/mL.

### Mode for Carrying Out the Invention

### Definition

As used herein, the term *"Camellia japonica"* is an evergreen tall tree that grows wild in China and Japan, as well as Korea, and up to about 15 m, with thick and glossy oval leaves. The fruits of *Camellia japonica* are formed after flowering, and the fruits completely ripen to generate seeds, from which camellia oil can be obtained by squeezing. In recent studies as well as *Donguibogam, Camellia japonica* leaves are effective for alleviating psoriasis, sore throat, and burns, and exerting antibacterial action; *Camellia japonica* branches and fruits are effective for scalp dandruff, chondrosis, menstrual disorders, diuresis, bleeding, hematemesis, and burns; and *Camellia japonica* flowers are traditionally known to be effective for gynecological diseases, for example, *Camellia japonica* flowers have been consumed as a tea to relieve extravasated blood.

As used herein, the term "influenza virus" is a causative virus of influenza, wherein virus particles have a coat and are spherical with a diameter of 80-120 nm, but exhibit polymorphism such as filamentous particles. A pathogen of influenza was discovered in 1933, and another virus thereof was discovered in 1940. Even people immune to the conventional virus are not immune to another virus, which causes influenza in the event of an infection. Therefore, to distinguish between the two, the virus discovered in 1933 was sorted as Type A and the virus discovered in 1940 was sorted as Type B. In 1944, an influenza virus different from Type A or Type B was discovered and named Type C.

### Detailed description:

In accordance with an aspect of the present disclosure, there is provided a pharmaceutical composition containing a *Camellia japonica* extract or an active fraction separated by chromatography of the *Camellia japonica* extract as an active ingredient for prevention and treatment of a viral infection.

The *Camellia japonica* extract may be an extract of a leaf, fruit, stems, or root part of *Camellia japonica,* preferably, an extract of a leaf or fruit of *Camellia japonica.*

In the pharmaceutical composition, the extract may be obtained by extraction with water, a C₁ to C₄ lower alcohol, or a mixture thereof, and the active fraction may be prepared by a preparative liquid chromatography process. The preparative liquid chromatography process may be high-performance liquid chromatography (HPLC) using a concentration gradient of a mixture solution of tertiary distilled water and methanol, as a developing solvent, and the active fraction may be a fraction eluted at a methanol concentration in the range of 30% to 70%, more preferably 40% to 60%, and most preferably 45% to 55%.

In the pharmaceutical composition, the virus may be an RNA virus, and the RNA virus may be a negative single stranded RNA virus, and the negative single stranded RNA virus may be a reovirus, a picornavirus, a calicivirus, a togavirus, an arenavirus, a flavivirus, an orthomyxovirus, a paramyxovirus, a bunyavirus, a rhabdovirus, a filovirus, a coronavirus, an astrovirus, a bornavirus, or an arterivirus. The orthomyxovirus may be an influenza A virus, an influenza B virus, an influenza C virus, influenza D virus, an isavirus, a thogotovirus, or a quaranjavirus. In addition, the influenza A virus may be human influenza A virus or avian influenza A virus.

In accordance with another aspect of the present disclosure, there is provided a health functional food containing a *Camellia japonica* extract or an active fraction separated by chromatography of the *Camellia japonica* extract as an active ingredient.

In accordance with another aspect of the present disclosure, there is provided use of a *Camellia japonica* extract or an active fraction separated by chromatography of the *Camellia japonica* extract for preparing a medicine for prevention and treatment of a viral infection.

In accordance with another aspect of the present disclosure, there is provided a method for treating a viral infection in a subject infected with a virus, the method including administering to the subject a therapeutically effective amount of a *Camellia japonica* extract or an active fraction separated by chromatography of the *Camellia japonica* extract.

In the treatment method, the treatment of the viral infection may be attained by inhibiting the expression of actin in an infected cell of the subject, inhibiting the replication of a virus in an infected cell of the subject, or inhibiting the release of virions from an infected cell of the subject.

In accordance with another aspect of the present disclosure, there is provided a method for preventing a viral infection in a subject, the method including administering to the subject a therapeutically effective amount of a *Camellia japonica* extract or an active fraction separated by chromatography of the *Camellia japonica* extract.

In accordance with another aspect of the present disclosure, there is provided a method for inhibiting the expression of actin in an infected cell in a subject, the method including administering to the subject a therapeutically effective amount of a *Camellia japonica* extract or an active fraction separated by chromatography of the *Camellia japonica* extract.

In accordance with another aspect of the present disclosure, there is provided a method for inhibiting the release of virions from an infected cell in a subject, the method including administering to the subject a therapeutically effective amount of a *Camellia japonica* extract or an active fraction separated by chromatography of the *Camellia japonica* extract.

The pharmaceutical composition of the present disclosure can be administered through oral administration or parenteral administration, and more specifically oral administration, but is not limited thereto. As for the parenteral administration, administration can be conducted through various routes, such as intravenous injection, intranasal inhalation, intramuscular administration, intraperitoneal administration, and transdermal absorption.

The pharmaceutical composition for treatment of liver disease of the present disclosure may be administered at a dose of 0.1 mg/kg to 1 g/kg, and more preferably at a dose of 1 mg/kg to 600 mg/kg. The dose may be appropriately adjusted according to the age, sex, and severity of a patient.

The pharmaceutical composition may be formulated with various formulations, for example, the pharmaceutical composition may be prepared as a decoction, which may be contained in a retort pouch, or may be formulated in the formulation of a power, a tablet, and a capsule after being dried through hot air drying or freeze drying, or may be formulated as a gel formulation by being mixed with a gelation ingredient, such as gelatin. Any known formulation that is used in the manufacture of pharmaceutical preparations may be used as needed.

In addition, one or more pharmaceutically acceptable carriers may be used for the preparation of the pharmaceutical composition of the present disclosure, and examples of these carriers may include, conventional organic or inorganic carries, such as an excipient, a lubricant, a binder, and a disintegrating agent when the composition is in a solid formulation, or a solvent, a solubilizing agent, an emulsifying agent, an isotonic agent, a buffer agent, and a soothing agent may be used when the composition is in a liquid formulation. Moreover, one or more additives, such as a conventional preservative, an antioxidant, a coloring agent, a sweetening agent, an absorbent, and a wetting agent, may be used as needed.

Furthermore, the heath functional food of the present disclosure may be a health functional food for alleviation or prevention of alcoholic fatty liver disease, wherein the health functional food is a preparation in the form of a capsule, a tablet, a powder, granules, a liquid, pills, flakes, a paste, a syrup, a jelly, or a bar. The health functional food may be used as various formulations suitable for a health functional food, for example, in the formation of a decoction, a drink, a powder, pills, a capsule, a tablet (a coated tablet, a sugarcoated tablet, a sublingual tablet, etc.), and a jelly.

The viral infection converts to optimize the normal functions of a host cell for virus replication and virion production. Above all, the structural change and reorganization of the intracellular actin are significantly converted to affect the entire life cycle including viral recombination and egression. Such cell transformation by viral infection is caused by Rous sarcoma viruses, avian retroviruses, simian virus 40, adenoviruses, and the like, and causes abnormal cell proliferation and morphological modification through several phases. Especially, actin plays an important role when the virus is exported out of the cell after the completion of the assembly of the virus in the host cell, and therefore, the egression of a whole virion can be inhibited by controlling the abnormal overexpression of actin. In the present disclosure, the *Camellia japonica* extract at a high concentration inhibits the expression of such intracellular actin very effectively, and thus can be developed as a novel material that prevents secondary infection of viruses.

For conventional treatment for viruses, an inhibitor for cell membrane fusion, an inhibitor for reverse transcriptase changing from RNA to DNA, a protease inhibitor that blocks the protein cleavage process of proteolytic enzymes, and the like were used, and in addition to these, an inhibitor for CCR5 receptor involved in binding at the stage of viruses fusing into host cells, an integrase inhibitor that inhibits becoming proviruses, a medicine that inhibits the final maturation stage in which newly created viruses egressed from the host cell, and the like were used as therapeutic agents. However, most of the medicines currently used, which are synthetic drugs and fail to completely suppress viruses, caused side effects, such as frequent drug resistances, reduced immunity, abnormal adipose distribution syndrome, mitochondrial toxicity, bone metabolism abnormality, and hepatotoxicity. Therefore, many studies are being conducted on the development of natural product-derived materials with few side effects and excellent antiviral effects. Since the fact that Tamiflu, which is used to treat novel swine-origin influenza A (H1N1), is derived from *Illicium verum* Hooker fil., the development of natural product drugs is expected to be made explosively. The present inventors realized that a natural product-derived material would have a very high value for use as an adjuvant therapy in combination with an antiviral agent when the material is confirmed to have two effects of controlling the decrease in immunity and suppressing viral amplification triggered by the RNA viral infection, and as a result of all efforts, developed a natural productpharmaceutical composition for prevention and treatment of a viral infection, the composition being extracted from leaves of *Camellia japonica* grown in Korea.

According to the present invention, it was confirmed that an active substance contained in the chromatographic fraction (F2) of the *Camellia japonica* extract has effects of inhibiting viral replication and suppressing the egression of a virus body, which has been completely assembled in a host cell, out of the cell. It was also confirmed that in an aspect of mechanism, the fraction (F2) inhibits normal production and extracellular release of viruses by specifically controlling the expression of the actin cytoskeleton, which is very important for the viral life cycle (attachment, invasion, nuclear localization, gene replication and reverse transcription, assembly, and egression and dissemination) in the host cell. It was also identified that the fraction (F2) contains hydroquinone, which is known to interfere with the primary viral infection and the attachment of the virus released from a host cell to a target cell, and thus the present inventive substance exhibits an effective antiviral effect by suppressing from the initial binding of a virus to the normal assembly and egression of the virus in an already-infected cell. The results as described above mean that the present inventive substance has a double anti-viral effect, which is more effective than that of Tamiflu, an anti-influenza medicine derived from *Illicium verum* Hooker fil. (inhibiting the egression of viruses) as a neuraminidase inhibitor, and thus can be developed as a next-generation new drug. Therefore, the present disclosure does not work specifically for a specific virus, but can be applied to various types of viruses.

Hereinafter, the present disclosure will be described in detail with reference to examples. However, the present disclosure is not limited to the examples described below, but may be implemented in various different forms. The following examples are provided to complete the present disclosure and to fully inform a person skilled in the art the scope of the present disclosure.

### Example 1: Preparation of Camellia japonica extract

In order to prepare a *Camellia japonica* extract, 100 g of completely dried and finely pulverized *Camellia japonica* leaves were immersed in 3 L of water, and then subjected to hot-water extraction at 95°C for 3 hours. The extract supernatant was collected and filtered through No. 1 filter paper. Thereafter, the filtered extract was freeze-dried to obtain 22.8 g of a final hot-water extract.

### Example 2: Separation and fractionation of Camellia japonica extract

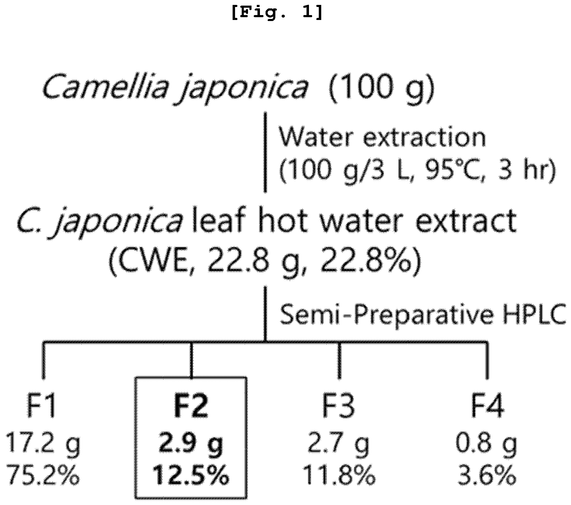

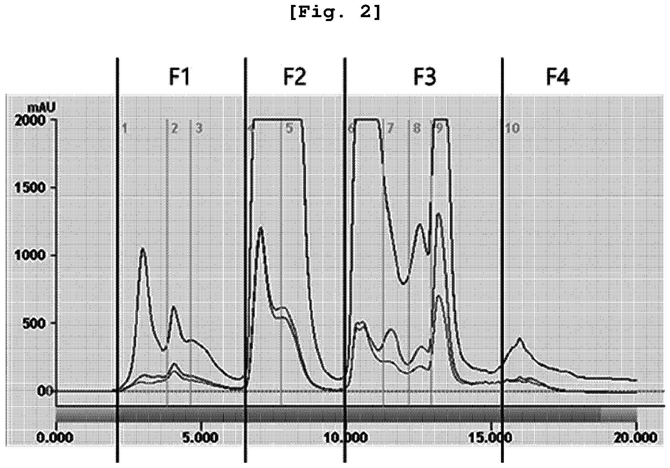

The *Camellia japonica* extract obtained in Example 1 was dissolved in tertiary distilled water to a concentration of 50 mg/mL, and then 10 mL was loaded on the YMC LC forte/R (YMC, Japan) HPLC device, followed by separation and fractionation (FIG. 1). Specifically, the flowing rate was 30 mL/min, and the absorbance was measured at wavelengths of 203 nm, 254 nm, and 280 nm. As for the column used for separation and fractionation, two YMC-DispoPackAT ODS-25/40g devices were serially connected and used for fractionation at room temperature. HPLC gradient conditions were as follows: solvent A was tertiary distilled water and solvent B was methanol; for separation of F1 to F4, F1 was separated in the 30% methanol concentration zone, F2 was separated in the 50% methanol concentration zone, and F3 and F4 were separated in the 10-16 minute zone and the 16-20 minute zone of the 70% or more methanol concentration, respectively, and analysis was performed under conditions where B%=10% at 0 minute, B%=10% at 0-3 minutes, B%=30% at 3-6 minutes, B%=50% at 6-9 minutes, B%=70% at 9-12 minutes, and B%=100% at 12-20 minutes (FIG. 2). As a result, as shown in FIGS. 1 and 2, the yield of the F1 fraction was highest (75.2%), and the yield of the F2 fraction was the next highest (12.5%), followed by F3 and F4 fractions.

### Example 3: GS-MS and TLC analysis

The gas chromatography-mass spectrometer (GC-MS) analysis and thin layer chromatography (TLC) analysis were performed on the obtained *Camellia japonica* hot-water extract (CWE) and the F2 fraction.

Specifically, the GC-MS analysis on the fraction and CWE having biological activity was performed using GC-MS (Agilent Technologies 5975C) equipped with CTC CombiPAL autosampler system (Palo, Alto, USA). The column used for sample analysis was HP-5 column (Agilent Technologies, 250 µm × 0.25 µm × 30 m), and the column was held at 50°C for 5 minutes, and the temperature of the column was raised by 10°C per minute, and then the column was held at 310°C for 5 minutes. Each sample was dissolved to 10 mg/mL using a suitable solvent for each for GC-MS analysis, and then 5 µL of each was injected by splitless injection. For TLC analysis, a hydroquinone standard (Sigma-Aldrich, USA), *Camellia japonica* hot-water extract (CWE), and the fraction (F2) were each dissolved in distilled water to a concentration of 10 mg/mL and dropped onto a TLC glass plate ten times. The TLC glass plate after dropping was put into a development tank, and then primary development was performed to 6 cm using methanol as a mobile phase. After the development site of 4 cm was cut off, secondary development was performed using ethyl acetate:methanol:water (77:13:10, v/v) as a mobile phase, resulting in a total of 9 cm of development. The developed glass plate was analyzed through iodine staining and ultraviolet irradiation (254 and 365 nm).

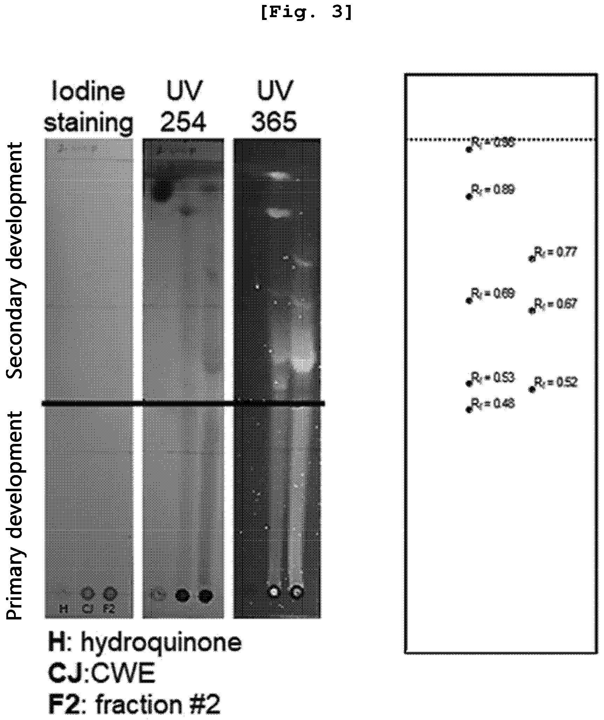

The GC/MC analysis results identified that hydroquinone (91-93% homogeneity), which is expected to have inhibitory activity against virus (HIV) and host cell membrane binding, was contained, wherein 11.9% and 5.5% of hydroquinone were contained in CWE and the F2 fraction, respectively. Although the content of hydroquinone contained in the F2 fraction was lower than that of CWE, a better effect was observed in the F2 fraction, and thus the above material was expected to a play a subsidiary role in terms of antiviral effect, but it was confirmed that the inhibition for the assembly and egression of the completed viral body (virion) essentially attributes to the antiviral effect of the active substance in the F2 fraction. Through the thin layer chromatography (TLC) analysis, hydroquinone (Rf=0.94) was confirmed at 254 nm in the F2 fraction fractionated from the *Camellia japonica* hot-water extract, and as a result at 365 nm, the F2 fraction showed a significantly high level of spot (Rf=0.52) compared with CWE, and the spot (Rf=0.74) existing only in the F2 fraction was identified (FIG. 3).

### Example 4: Inhibition of cytoskeletal actin gene expression

The viral infection converts to optimize the normal functions of a host cell for virus replication and virion production. Above all, the structural change and reorganization of the intracellular actin are significantly converted to affect the entire life cycle including viral recombination and egression. Such cell transformation by viral infection is caused by Rous sarcoma viruses, avian retroviruses, simian virus 40, adenoviruses, and the like, and causes abnormal cell proliferation and morphological modification through several phases. Especially, actin plays an important role when the virus is exported out of the cell after the completion of the assembly of the virus in the host cell, and therefore, the egression of whole virions can be inhibited by controlling the abnormal overexpression of actin.

Therefore, the effects of inhibiting the expression of cytoskeletal actin gene and inhibiting the release of virions, which were formed in host cells infected with viruses to cause secondary infection in neighbor cells, according to the treatment with the *Camellia japonica* extract (CWE) of the present disclosure were analyzed using reverse transcription-polymerase chain reaction (RT-PCR).

Specifically, in order to investigate whether the cytoskeletal actin gene expression was inhibited, a mouse macrophage cell line (Raw264.7) was treated with the *Camellia japonica* hot-water extract (CWE) and fractions (F1-F4) of the present disclosure. For culturing of the cells, alpha minimal essential media (Hyclone, UT, USA) containing 10% fetal bovine serum were used, and when the growth of the cells reached 90% in an incubator adjusted to 37°C and 5% CO₂, the cells were used for tests. The passage was controlled not to exceed 20 times. Thereafter, the cultured cells were suspended in 0.25% trypsin-EDTA, and then counted using a hemocytometer. For the cytotoxicity test, the cultured cells were seeded at 1.0×10⁴ cells/well in a 96-well plate. Furthermore, as for the gene expression test, the cells were seeded at 1.5×10⁶ cells/well in a 6-well plate, and after 2 hours of pre-culture, the cells were used for the actin gene expression test.

The expression level of the actin gene was quantified through RT-PCR. For performing this, first, total RNA of the cultured cells was extracted from Raw264.7 cells by using Trizol reagent (Invitrogen, MA, USA). That is, the cells were lysed by addition of 1 mL of Trizol reagent, and left at room temperature for 5 minutes, and then 200 µL of chloroform was added, followed by centrifugation at 13,500 rpm for 15 minutes. Thereafter, the transparent supernatant (500 µL) was taken out and transferred to a new tube, and an equal amount of isopropyl alcohol was added, followed by centrifugation at 13,500 rpm for 10 minutes, thereby precipitating RNA. The RNA precipitate was washed with 0.75 mL of 70% ethanol diluted in distilled water and treated with diethyl pyrocarbonate (DEPC, Sigma-Aldrich), dried in air, and used as a sample for reverse transcription. In addition, the total RNA was treated with the *Camellia japonica* hot-water extract of the present disclosure at different concentrations (10 to 100 µg/ml), and the total RNA treated with 1 mL of hydrogen peroxide (H₂0₂) was used as a control. The primary DNA strand cDNA synthesis was performed using 1 µg of the extracted total RNA, and the reverse transcription was performed using the Improm-II reverse transcription system (Promega) and oligo dT primers (see Table 1). In addition, PCR was performed through the 35-cycle chain reaction using 2x premix (ReddyMix PCR Master Mix, CA, USA) containing tag-polymerase. The nucleic acid sequences of the primers used in PCR are summarized in Table 1 below.

**TABLE 1**

| Primer | Nucleic acid sequence (5'--> 3') | SEQ ID NO |
|---|---|---|
| Forward | TACAGCTTCACCACCACAG C | 1 |
| Reverse | AAGGAAGGCTGGAAAAGAG C | 2 |

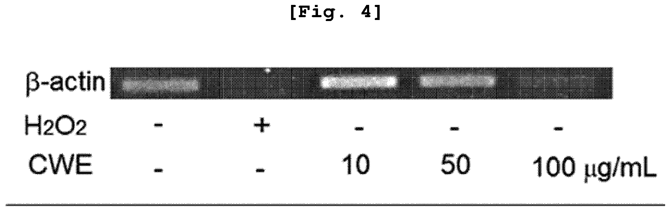

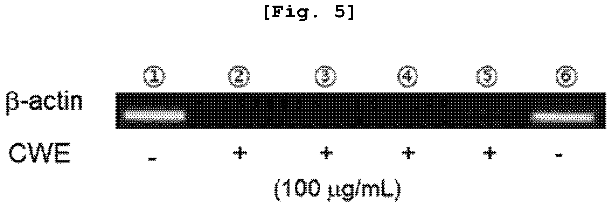

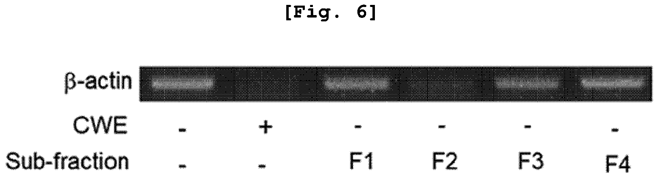

As a result, it was confirmed that the expression of actin was inhibited when the cells were treated with 100 µg/ml the *Camellia japonica* hot-water extract of the present disclosure (FIG. 4). In the gene amplification, among the non-treatment group (①), the treatment of cDNA with CWE dissolved in ethanol followed by PCR (②), the treatment of cDNA with CWE dissolved in distilled water followed by PCR (③), the treatment of total RNA with CWE, cDNA procedure, followed by PCR (④), the treatment of total RNA with CWE, the treatment of cDNA with CWE, followed by PCR (⑤), and the treatment of total RNA with distilled water, the treatment of cDNA with distilled water, followed by PCR (⑥), the expression of actin was inhibited in all the groups except for the negative groups ① and ⑥, and thus the inhibition of actin gene expression by the *Camellia japonica* extract of an embodiment of the present disclosure appeared to act on both two reactions, reverse transcription and DNA polymerization (FIG. 5). The above results are considered to suppress to the egression of virions through the inhibition of the abnormal overexpression of actin in the event of viral infection. As for the actin gene expression control effect of the fractions (F1 to F4) fractionated from the *Camellia japonica* hot-water extract (CWE) of the present disclosure, a similar activity was confirmed in the F2 fraction with a lower concentration than the CWE, and thus, it was confirmed that the active substance contained in the F2 fraction showed a direct antiviral effect (FIG. 6) .

### Example 5: Immunocytochemical staining analysis

In order to compare the protein expression level of actin, Raw264.7 cells were treated with the F2 fraction at 1, 25, and 50 µg/mL, and then subjected to immunocytochemical staining.

Specifically, for immunocytochemical analysis, the Raw264.7 cells cultured in a slide culture chamber were fixed in 4% paraformaldehyde for 15 minutes and washed with a phosphate buffer (PBS) supplemented with 100 mM glycine for 5 minutes, followed by the addition of 0.1% Triton X-100 (Sigma-Aldrich) solution as a cell membrane penetration solution, and then the cells were left at room temperature for 30 minutes. The cells were blocked by the treatment with 1% bovine serum albumin, followed by incubation at room temperature for 30 minutes, and then washed three times with a phosphate buffer. Then, the beta-actin monoclonal antibody was diluted 1:200 in the Tween 20-phosphate buffer containing 1% bovine serum albumin, followed by an antigen-antibody reaction at room temperature for 3 hours, and then washing was conducted. Thereafter, the cells were reacted with the secondary antibody conjugated with Alexafluor-594 attached thereto for 2 hours, followed by observation using a fluorescence microscope.

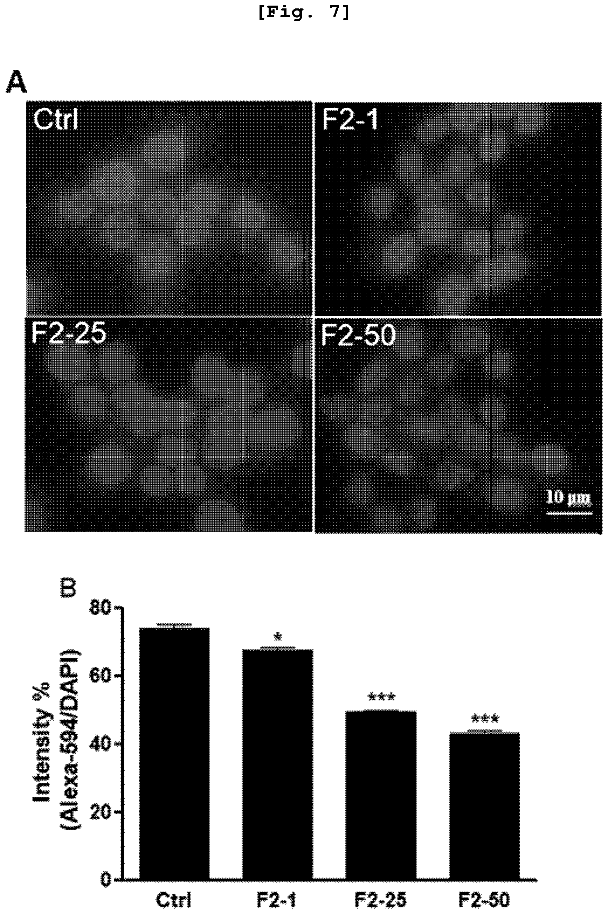

As a result, the entire distribution of cytoskeletal actin was low, and these results mean that the actin gene control by the *Camellia japonica* hot-water extract was effective (FIG. 7).

### Example 6: Inhibition of virion release

To analyze the viruses that were egressed from packaging cells (GP2-293) by the *Camellia japonica* extract and the active fraction (F2) according to an embodiment of the present disclosure, the GP2-293 culture media was collected to concentrate the viruses, and then viral PCR was performed using primers for the viral vector gene.

Specifically, a commercially constructed virus infection system (pQCXIP Retroviral Vector, Clontech, CA, USA) was used to investigate the virion release inhibitory effect of the *Camellia japonica* extract. That is, a viral infection-like test was performed by configuring a cell line transformed to stably perform the production of the MMLV Gag-Pol and pCMV-VSVG viral expression vectors and virions, which were required for viral packaging. First, the GP2-293 packaging cells (derived from human embryonic kidney cells HEK 293) expressing intracellular gag and pol proteins were co-transfected with Retro-x vector and pVSVG (envelop plasmid) to produce viral proteins and to replicate viral RNA in the GP2-293 cells. The virions in which the viral genes and viral proteins were assembled were allowed to move to the cell membrane, and the infectious virions were allowed to release (egress) of the cell. In the above procedure, the gene expression inhibition level of the *Camellia japonica* extract was compared by comparing the expression of actin between the normal cell line HEK 293 cells and the transformed GP2-293 cells. To investigate the presence or absence of the egressed viruses, the antiviral effect of the *Camellia japonica* extract was investigated through RNA gene qualitative analysis.

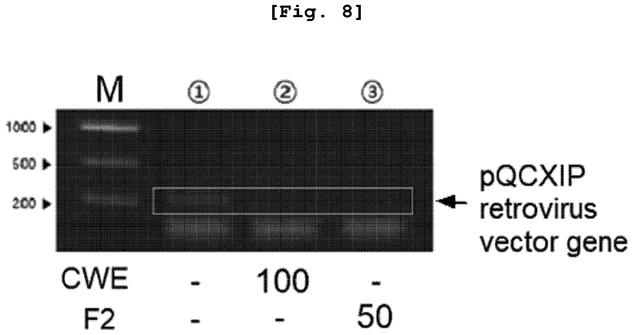

As a result, the RNA viral vector gene (194 base pairs, 5' primer: 2141-2164; 3' primer: 2329-2352) was not amplified in both the test groups treated with CWE (100 µg/mL) and the F2 fraction (50 µg/mL), and thus secondary infection by viruses was effectively controlled in the vicinity of virus-infected cells (FIG. 8).

### Example 7: Antiviral effect test

To search the antiviral effect, a cytopathic effect (CPE) inhibition test and an MTT (3-(4,5-dimethythiazol-2-yl)-2,5-diphenyl tetrazolium bromide) assay were performed using sensitive cells *in vitro.* The antiviral agents, amantadine (M2 protein inhibitor) and ribavirin (RNA synthesis inhibitor), were used as positive controls. The order of tests was cell adhesion, the analysis of cytotoxicity and antiviral activity against viruses (Flu A 2 types; PR8/Hong Kong, Flu B 1 type; Lee) for the cytopathic effect (CPE) test, and then the investigation of antiviral activity using the MTT assay. Madin-Darby Canine Kindey (MDCK) cells were used as sensitive cells for culturing each virus, and a minimum essential medium (MEM) containing 10% fetal bovine serum and penicillin/streptomycin/nystatin at 1% for each was used as a medium for maintaining and culturing MDCK cells.

The number of seeded MDCK cells was maintained at 2.0×10⁵ cells/well, and the serum-free MEM containing a vitamin solution, diglucose, and trypsin was used as a medium for virus infection. The viruses for infection were cultured in a 75-cm² tissue culture flask. When the adhering cells fell out due to the occurrence of CPE, freezing-thawing was repeated twice, followed by centrifugation at 1,500×g for 5 minutes, and the cell residue was discarded, and then the supernatant containing viruses was dispensed into frozen vials and stored at - 80°C for use. As for the infectious titer of the viruses for infection, the 50% cell culture infection dose (CCID₅₀) was calculated, and 30,000-fold dilutions of the crude cultures (1.2, 0.6, 3.8) of each of Flu A (PR8, Hong Kong) and Flu B (Lee) were used. As for the antiviral effect test for the *Camellia japonica* extracts (CWE and F2), the host cells were cultured under the same conditions as in the cytotoxicity test, and then the medium for culturing was discarded, and 100 µL of a culture medium for infection containing each extract at the 3-fold serial dilution concentration and 100 µL of viruses having 10 times the amount of TCID₅₀ were infected. After culturing for 2 days, the CPE inhibitory effects of the extracts were compared and observed contrary to the cytotoxicity test. The CPE inhibitory effect was compared and analyzed by placing the untreated cells, the cells infected with three types of viruses, and the cells with *Camellia japonica* extracts (CWP and F2) added thereto, and the comparative analysis was repeated twice per sample while the controls were used.

As a result, as shown in Table 2 below, in the CC₅₀(µg/mL) analysis, the cytotoxicity of each of the *Camellia japonica* extracts (CWE and F2) was >2,400 µg/mL, indicating no cytotoxicity on test cells MDCK. Therefore, as a result of observing the anti-influenza effect assay of CWE and F2 by applying the method for determining the inhibition of CPE, induced from viral infection, CWE and F2 fraction were observed to have excellent antiviral activity, and in particular, the F2 fraction showed high antiviral activity against Flu A and Flu B, and also showed high selectivity index (SI), as analyzed by CC₅₀/EC₅₀ compared with the positive controls, confirming excellent antiviral activity.

**TABLE 2**

| Antiviral activity comparison test results | | | | | | | | |
|---|---|---|---|---|---|---|---|---|
| NO | Code | Toxicity CC₅₀ (ug/ml) | Antiviral activity (EC₅₀: ug/ml) | | | Selectivity index | | |
| | | | FluA | FluA | FluB | FluA | FluA | FluB |
| | | | H1N1 | H3N2 | - | H1N1 | H3N2 | - |
| | | | PR8 | HongKong | Lee | PR8 | HongKong | Lee |
| 1 | CWE (µg) | >2400.0 | 31.4 | <29.6 | 74.0 | >76.4 | >81.1 | >32.4 |
| 2 | F2 (µg) | >2400.0 | <29.6 | <29.6 | 45.7 | >81.1 | >81.1 | >52.5 |
| 4 | Solvent (methanol) | >42.0 | >42.0 | >42.0 | >42.0 | ND | ND | ND |
| 5 | AMT (µM) (Amantadin) | >100.0 | >100.0 | 2.3 | >100.0 | ND | >43.5 | ND |
| 6 | RBV (µM) (Ribavirin) | >100.0 | 29.9 | 13.4 | 19.6 | >3.3 | >7.5 | >5.1 |

In conclusion, according to the pharmaceutical composition containing the *Camellia japonica* extract for prevention and treatment of a viral infection of the present disclosure, the extract obtained by extraction from the leaves of *Camellia japonica* was observed to have an excellent antiviral effect, such as controlling gene amplification through the inhibition of viral reverse transcription and inhibiting the expression of the cytoskeletal (actin) protein essential for viral egression, without affecting the activity and survival of infected target cells, and thus can be developed as a material for natural product drugs that prevent secondary viral infection.

The present disclosure has been described with reference to the embodiments, but these are intended to be merely illustrative, and a person skilled in the art will understand that various modifications and other equivalent embodiments can be derived therefrom. Accordingly, the true scope of protection of the present disclosure should be defined based on the appended claims.

## Claims

1. A pharmaceutical composition for prevention and treatment of a viral infection containing a *Camellia japonica* extract or an active fraction separated by chromatography of the *Camellia japonica* extract as an active ingredient.

2. The pharmaceutical composition of claim 1, wherein the extract is obtained by extraction with water, a C₁ to C₄ lower alcohol, or a mixture thereof.

3. The pharmaceutical composition of claim 1, wherein the active fraction is prepared by a preparative liquid chromatography process.

4. The pharmaceutical composition of claim 1, wherein the virus is a RNA virus.

5. The pharmaceutical composition of claim 4, wherein the RNA virus is a negative single-stranded RNA virus.

6. The pharmaceutical composition of claim 5, wherein the negative single-stranded RNA virus is a reovirus, a picornavirus, a calicivirus, a togavirus, an arenavirus, a flavivirus, an orthomyxovirus, a paramyxovirus, a bunyavirus, a rhabdovirus, a filovirus, a coronavirus, an astrovirus, a bornavirus, or an arterivirus.

7. The pharmaceutical composition of claim 6, wherein the orthomyxovirus is an influenza A virus, an influenza B virus, an influenza C virus, influenza D virus, an isavirus, a thogotovirus, or a quaranjavirus.

8. An antiviral health functional food containing a *Camellia japonica* extract or an active fraction separated by chromatography of the *Camellia japonica* extract as an active ingredient.

9. Use of a *Camellia japonica* extract or an active fraction separated by chromatography of the *Camellia japonica* extract for preparing a medicine for prevention and treatment of a viral infection.

10. A method for treating a virus in a subject infected with the virus, the method comprising administering to the subject a therapeutically effective amount of a *Camellia japonica* extract or an active fraction separated by chromatography of the *Camellia japonica* extract.

11. A method for preventing a viral infection in a subject, the method comprising administering to the subject a therapeutically effective amount of a *Camellia japonica* extract or an active fraction separated by chromatography of the *Camellia japonica* extract.

12. A method for inhibiting the expression of actin in an infected cell in a subject infected with a virus, the method comprising administering to the subject a therapeutically effective amount of a *Camellia japonica* extract or an active fraction separated by chromatography of the *Camellia japonica* extract.

13. A method for inhibiting the release of virions from an infected cell in a subject infected with a virus, the method comprising administering to the subject a therapeutically effective amount of a *Camellia japonica* extract or an active fraction separated by chromatography of the *Camellia japonica* extract.
